(19) Europäisches Patentamt
European Patent Office
Office européen des brevets

(11) Publication number: 0 152 799
B1

(12) EUROPEAN PATENT SPECIFICATION

(45) Date of publication of patent specification: 28.10.87

(51) Int. Cl.$^4$: C 07 D 295/08, A 61 K 31/495

(21) Application number: 85100737.7

(22) Date of filing: 25.01.85

# (54) Compounds having peripheral calcium antagonist, anticonvulsive and eumetabolic activity, processes for the preparation thereof and pharmaceutical compositions containing them.

(30) Priority: 02.02.84 IT 1937284

(43) Date of publication of application:
28.08.85 Bulletin 85/35

(45) Publication of the grant of the patent:
28.10.87 Bulletin 87/44

(84) Designated Contracting States:
AT BE CH DE FR GB IT LI LU NL SE

(56) References cited:
FR-A-2 159 369
GB-A-1 268 710

(73) Proprietor: PULITZER ITALIANA S.p.A.
Via Tiburtina, 1004
I-00156 Rome (IT)

(72) Inventor: Quadro, Giuseppe
Via Pisacane, 34/A
I-20129 Milan (IT)

(74) Representative: Bianchetti, Giuseppe
Studio Consulenza Brevettuale Via Rossini, 8
I-20122 Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 152 799 B1

Courier Press, Leamington Spa, England.

**Description**

The present invention relates to 1-(4,4'-difluoro-benzhydryl)-4-(3,4-dimethoxycinnamyl)-piperazine of the formula I

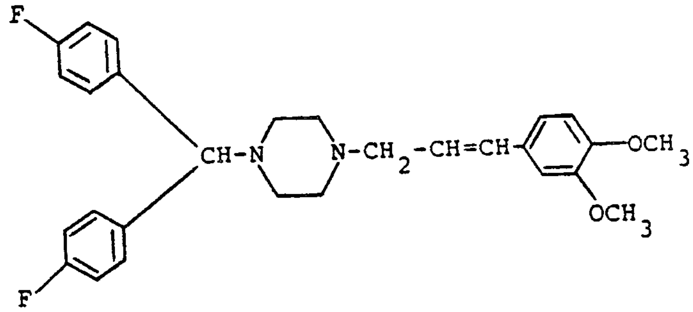

(I)

and pharmaceutically acceptable acid addition salts thereof.

Compound I exhibits potent peripheral calcium antagonist, anticonvulsive and eumetabolic activities, together with a very low toxicity, and therefore it may be employed in the treatment of circulatory diseases, convulsions and cerebral circulatory diseases.

According to the present invention, compound I may be used in therapy in form of the free base as well as in form of pharmaceutically acceptable salts thereof with inorganic or organic acids. Among the salts, particularly preferred is the dihydrochloride which will be hereinafter called, for sake of shortness, also with abbreviations PU 122.

The present invention also relates to pharmaceutical compositions containing as the active ingredient the compounds of the invention, optionally in admixture with suitable conventional excipients. Said pharmaceutical compositions may be administered orally, parenterally or rectally, using conventionally employed carriers, excipients, lubricants, emulsifiers, flavouring and colouring agents, etc.

Compound I may be prepared by reacting 1-(4,4'-difluoro-benzhydryl)piperazine with a reactive derivative of 3,4-dimethoxycinnamic acid, such as its acyl halide, symmetric (homogeneous) or mixed anhydride in presence of a tertiary organic base and subsequently treating the resulting amide with a suitable reducing agent, which may be a metallic hydride such as lithium aluminium hydride or sodium borohydride.

Alternatively, compound I may be prepared by condensing 1-(4,4'-difluoro-benzhydryl)piperazine with a cinnamyl halide in the presence of an organic or inorganic base, for example a tertiary organic base.

The following examples further illustrate the invention, without limiting in any way the scope thereof.

Example 1

a) 3,4-Dimethoxycinnamoyl chloride

3,4-Dimethoxycinnamic acid (9.85 g; 0.05 mole) was reacted with thiony chloride (50 ml). The reaction mixture was boiled under stirring for 5 hours, the the excess thionyl chloride was evaporated under reduced pressure and a dark residue was obtained which was take up in anhydrous benzene (50 ml) and evaporated under reduced pressure. Such a treatment was repeated one more time, and the resulting residue was employed in the subsequent step, optionally after crystallization from ligroine (7.0 g; 0.031 mole; 61.7% yield). M.p. 82—83°C (not corrected).

b) 1-(4,4'-Difluorobenzhydryl)-4-(3,4-dimethoxycinnamoyl)piperazine

1-(4,4'-Difluorobenzhydryl)piperazine (7.1 g; 0.0247 mole) was dissolved in anhydrous toluene (75 ml); then anhydrous triethylamine (2.5 g; 3.5 ml; 0.0247 mole) was added and the reaction mixture was cooled to about 5°C, thereafter the previously prepared acyl chloride (5.6 g; 0.0247 mole) diluted in anhydrous toluene (30 ml) was added, under slow stirring. After completion of the addition, the temperature was left raise to the room temperature, then the reaction mixture was gradually heated to reflux, which was kept for about 4 hours. The course of the reaction, during which triethylamine hydrochloride and part of the desired amide separated, was checked by TLC.

After cooling, the reaction mixture was evaporated and the residue was treated with water (200 ml). The resulting aqueous emulsion was extracted with dichloromethane (200 ml × 3), the organic layer was washed in turn with water, 5% aqueous sodium hydroxide, water, then it was dried over $Na_2SO_4$, decolorized with active carbon, filtered and evaporated, to obtain the desired amide, which was crystallized from ethanol (800 ml) to give the pure product (5.5 g; 0.01 mole; 46.5% yield), melting at 220—222°C (not corrected).

Elemental analysis: $C_{28}H_{28}F_2N_2O_3$ (p.m. 478.55)
Calc.% = C 70.28 H 5.90 N 5.85
Found% = C 70.12 H 5.88 N 5.90

TLC = silica gel plates; eluent absolute EtOH; U.v. light = unitary spot; $R_f$ = 0.78.

c) 1-4,4'-Difluorobenzhydryl)-4-(3,4-dimethoxycinnamyl)piperazine dihydrochloride

To a suspension of aluminium-lithium hydride (1.3 g) in anhydrous tetrahydrofuran (300 ml) a solution of the amide from step b) (16.5 g; 0.034 mole) in anhydrous tetrahydrofuran (800 ml) was slowly added; after that the reaction mixture was stirred for about 8 hours, then it was treated in turn with ethyl acetate, water, 20% aqueous sodium hydroxide, and water. The alakali hydrates were filtered off and the filtrate was evaporated under reduced pressure, to give an oily residue which was dissolved in dichlorometane. The resulting solution was washed in turn with water, 5% aqueous sodium hydroxide and water; dried on $K_2SO_4$, filtered and evaporated to give an oily residue (17 g; 0.036 mole) which was salified in an ethanol-ether mixture by addition of hydrochloric acid dissolved in ethanol.

The precipitated raw salt was crystallized from ethanol (300 ml) to chromatographic purity (5.0 g; 0.0093 mole; 27.5% yield).

Elemental analysis: for $C_{28}H_{32}Cl_2F_2N_2O_2$ (p.m. 537.49)
Calc.% = C 62.57 H 6.00 N 5.21
Found% = C 62.52 H 6.13 N 5.17
M.p. = 231—233°C (not corr.)
TLC = silica gel plates-eluent absolute EtOH — U.V. light — unitary spot $R_f$ = 0.65.

Example 2

a) 1-(4,4'-Difluorobenzhydryl)-4-(3,4-dimethoxycinnamoyl)piperazine

3,4-Dimethoxycinnamic acid (9.85 g; 0.05 mole) was dissolved in anhydrous acetone (100 ml) after that anhydrous triethylamine (5.56 g; 7.6 ml; 0.055 mole) was added. The reaction mixture was cooled to 0—5°C, then ethyl chlorocarbonate was added. The mixture was stirred at the same temperature, for about 20 minutes, during which time the triethylamine hydrochloride separated, the mixed anhydride forming in the solution. 1-(4,4'-Difluorobenzhydryl)piperazine (14.4 g; 0.05 mole) was separately dissolved in anhydrous acetone (80 ml; and the resulting solution was added in one portion to the flask containing the mixed anhydride. The mixture was stirred for 60 minutes at about 0—5°, then for about 4 hours, allowing the temperature to raise to the room temperature. The reaction was checked by TLC (abs. EtOH). After completion of the reaction, the solution was filtered and the filtrate was evaporated under reduced pressure, to give a residue which was treated with dichloromethane. The resulting solution was washed in turn with water, 5% aqueous sodium hydroxide, water, 1% aqueous hydrochloric acid and water. The organic solution was dried over $K_2SO_4$, filtered and evaporated to give the desired salt (18.9 g; 0.039 mole; 79% yield) which was crystallized from ethanol. The obtained product was the same as the one of example Ib) (m.p.; mixed m.p.; TLC).

b) 1-(4,4'-Difluorobenzhydryl)-4-(3,4-dimethoxycinnamyl)piperazine dihydrochloride

The amide from step a) was reduced and worked out as described in Example Ic) to give the title compound, which was the same as the one obtained according to Example I (m.p.; mixed m.p.; TLC).

Example 3

1-(4,4'-Difluorobenzhydryl)-4-(3,4-dimethoxycinnamyl)piperazine dihydrochloride

1-(4,4'-Difluorobenzhydryl)piperazine (32.3 g; 0.112 mole), 3,4-dimethoxycinnamyl chloride (23.82 g; 0.112 mole) and potassium carbonate (15.5 g; 0.112 mole) were mixed in 1-butanol (100 ml); the reaction mixture was boiled to reflux for 9 hours. The course of the reaction was checked by TLC (abs. EtOH). After cooling, the inorganic salts were filtered off and washed on the filter with 1-butane; then filtrate and washings collected were evaporated to dryness under reduced pressure. The resulting oily residue was treated with dichloromethane, and the organic solution was washed in turn with water, 5% aqueous sodium hydroxide and water, dried over $K_2CO_3$, filtered and evaporated under reduced pressure, to give oily residue (19.2 g; 0.041 mole; 36.9% yield) which was salified and crystallized, as described in Example 1c). The bishydrochloric salt obtained was the same as in the above examples (m.p.; mixed m.p.; TLC).

Example 4

1-(4,4'-Difluorobenzhydryl)-4-(3,4-dimethoxycinnamyl)piperazine dihydrochloride

1-(4,4'-Difluorobenzhydryl)-4-(3,4-dimethoxycinnamoyl)piperazine (2.87 g; 0.006 mole) obtained as described in Example 1b) was dissolved in tetrahydrofuran (20 ml) and the solution was treated with sodium borohydride (1.2 g; 0.031 mole). The reaction mixture was cooled to about 10°C, then trifluoroacetic acid (2.3 ml; 0.031 mole) diluted with tetrahydrofuran (10 ml) was added dropwise during a hour.

The mixture was refluxed for 48 hours under stirring, then, after cooling, the excess reactive was destroyed by acidification with 10% aqueous hydrochloric acid. The reaction mixture was filtered and the filtrate was evaporated under reduced pressure. The resulting residue was crystallized to purity from ethanol, obtaining the title compound (0.6 g; 19% yield) which was the same as the one obtained in Example 1c).

Compound PU 122 proved to have a vasodilating activity higher than that of Flunarizine, a well known vasodilative agent, used as a comparison. Moreover, PU 122 shows an interesting antiaggregating activity higher than that of the acetylsalicylic acid.

Cerebral perfusion in the rabbit

The cerebral vasodilative activity of PU 122 in comparison with Flunarizine was tested by the method of the cerebral perfusion in the rabbit according to the procedure of Von P. Vaupel e H. Hutten (1980), by measurement of the decreases of the peripheral resistances in the cerebral district.

Said changement was determined by means of a transducer, by measuring the hematic pressure at the cerebral level and scoring the lasting of action of the compound under test.

The two test compounds were administered by intraarterial route at the dosage of 0.9 µM, in a volume of 0.2 ml for each animal.

The results are reported in the following Table 1, which shows how PU 122 has a marked vasodilative activity at the cerebral level, substantially higher than that of Flunarizine.

TABLE 1

Cerebral vasodilative activity in the rabbit of PU 122 and Flunarizine.
Perfusion of the cerebral district.

| Treatment | Doses uM i.a. | No. of animals | Difference in pressure | Δ | Lasting of the effect (min.) | Area $mm^2$ |
|---|---|---|---|---|---|---|
| Flunarizine | 0.9 | 5 | 10.25 | 25.12 | 12.50 | 314.2 |
| PU 122 | 0.9 | 5 | 18.75 | 29.17 | 16.70 | 487.14 |

Rat hind quarters

The vasodilative activity of PU 122 in comparison with Flunarizine was further evaluated according to the procedure described by F. N. Fastier and F. H. Smirk, (J. Pharm. Exp. Therap. *89*, 256—270; 1974), by measuring the antagonist action of the test compound againt hyperkaliemia induced vasoconstriction.

The compounds under test were used at $5.10^{-5}$ M concentration. The results are reported in the following Table 2, which shows how both the test compounds have high vasodilative activity, PU 122 being markedly more active.

TABLE 2

Vasodilative activity of PU 122 and Flunarizine. Rat hind quarters perfused
with hyperkaliemic tyrode.

| Treatment | Concentration | No. of animals | Perfusion pressure | | | Δ% |
|---|---|---|---|---|---|---|
| | | | basal | after 10′ | Δ | |
| PU 122 | $5 \times 10^{-5}$ M | 5 | 195.00 | 90.00 | 105.00 | 53.85 |
| Flunarizine | $5 \times 10^{-5}$ M | 5 | 175.00 | 95.00 | 80.00 | 45.71 |

$Ca^{++}$ antagonist activity

The $Ca^{++}$ antagonist activity of PU 122 in comparison with Nifedipine was evaluated by measurement of the 45 $Ca^{++}$ uptake, according to the procedure of Deana et al. (Biochem. J., 218, 899—905, 1984), in synaptosomial preparations of rat brain, obtained according to the method of Booth and Clark (Biochem. J. 176, 365—370, 1978). The results are reported in Table 3, which show how PU 122 has a remarkable inhibitory effect on the $Ca^{++}$ uptake in rat brain synaptosomes, even though lower than that of Nifedipine as a standard control.

TABLE 3

$Ca^{++}$ antagonist activity

| | |
|---|---|
| PU 122 | −16.4% |
| Nifedipine | −50% |

Platelet antiaggregating activity

The inhibitory effect on platelet aggregation induced BY ADP on human platelet rich plasma (PRP) was evaluated by PU 122 in comparison with acetylsalicylic acid, according to the procedure described by G.V.R. Born (Nature, *194,* 927; 1962).

The obtained results proved how PU 122 has a strong platelet antiaggregating activity with a $IC_{50}$ substantially better than that of acetylsalicylic acid used as the reference drug.

The present invention refers also to all the industrially applicable aspects connected with the therapeutic use of compound I or of its salts. An essential aspect of the invention is therefore provided by pharmaceutical compositions containing, as active principle, predetermined and therapeutically effective amounts of compound I or of its salts, in addition with carriers conventionally used in pharmaceutical preparations. Examples of such pharmaceutical formulations comprise capsules, tablets, dragees, granulates, lozenges, sugar coated tablets, syrups, solutions, suppositories, vials for intromuscular or intravenous injection, containing from 1 to 250 mg of active principle and can be administered 1 or more times a day according to the diagnosis and weight and age of the patient.

**Claims for the Contracting States: BE CH DE FR GB IT LI LU NL SE**

1. 1-(4,4'-Difluorobenzhydryl)-4-(3,4-dimethoxycinnamyl)piperazine of the general formula

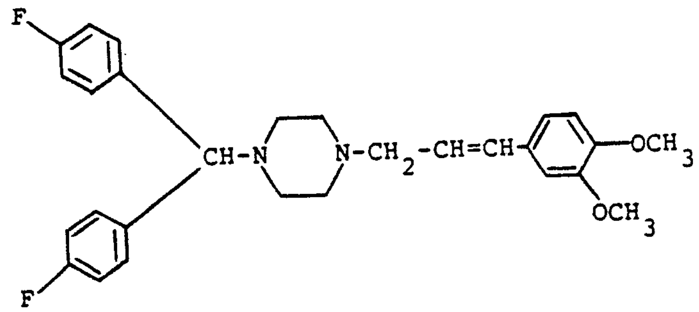

(I)

and its pharmaceutically acceptable salts with organic or inorganic acids.

2. As a compound accordingd to claim 1, 1-(4,4'-difluorobenzyhydryl)-4-(3,4-dimethoxycinnamyl)piperazine dihydrochloride.

3. A process for the preparation of compounds according to claims 1—2, characterized by reacting a reactive derivative of 3,4-dimethoxycinnamic acid with 1-(4,4'-difluorobenzhydryl)piperazine and subsequently reducing the resulting compound by means of reducing agents such as metallic hydrides.

4. Pharmaceutical compositions having peripheral calcium anatagonist, anticonvulsive and cerebral eumetabolic activity, containing as the active principle the compounds according to claims 1—2.

5. Pharmaceutical compositions according to claim 5, in form of capsules, tablets, dragees, syrups, solutions, granulates, lozenges, vials.

**Claims for the Contracting State: AT**

1. Process for the preparation of 1-(4,4'-Difluorobenzhydryl)-4-(3,4-dimethoxycinnamyl)piperazine of general formula

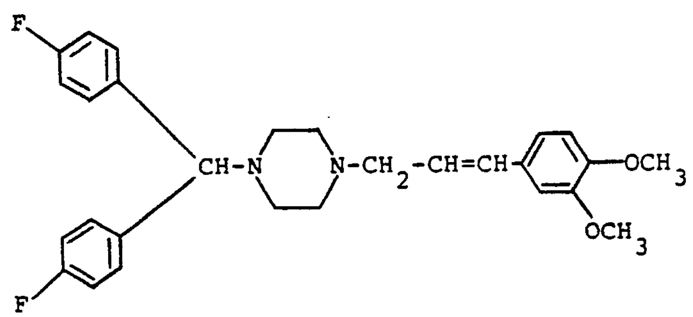

(I)

characterized by reacting a reactive derivative of 3,4-dimethoxycinnamic acid with 1-(4,4'-difluorobenzhydryl)piperazine and subsequently reducing the resulting compound by means of reducing agents.

2. Process according to claim 1 characterized in that the reaction is carried out in the presence of a tertiary organic base.

3. Process according to claim 1 wherein the reduction is carried out by means of metal hydrides such as lithium aluminium hydride or sodium borohydride.

4. Process for the preparations of 1-(4,4'-difluorobenzhydryl)-4-(3,4-dimethoxycinnamyl)piperazine characterized by reacting 3,4-dimethoxycinnamyl halide with 1-(4,4'-difluorobenzhydryl)piperazine in the presence of a tertiary organic base.

**Patentansprüche für die Vertragstaaten: BE CH DE FR GB IT LI LU NL SE**

1. 1-(4,4'-Difluorbenzhydryl)-4-(3,4-dimethoxycinnamyl)piperazine der allgemeinen Formel I

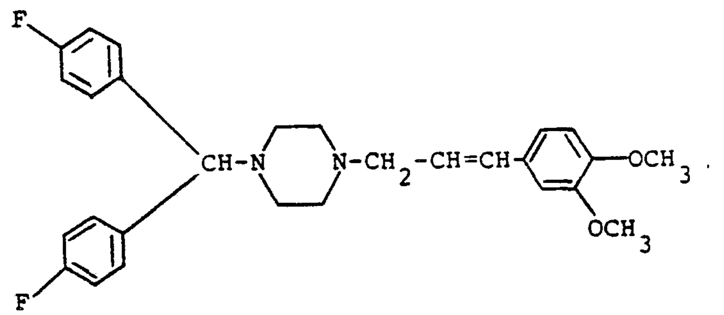

(I)

und dessen pharmazeutisch unbedenkliche Salze mit organischen oder anorganischen Säuren.

2. Verbindung nach anspruch 1, 1-(4,4'-Difluorbenzhydryl)-4-(3,4-dimethoxycinnamyl)piperazin-dihydrochlorid.

3. Verfahren zur Herstellung von Verbindung nach den Ansprüchen 1 bis 2, dadurch gekennzeichnet, daß ein reaktives Derivat von 3,4-Dimethoxyzimtsäure mit 1-(4,4'-Difluorbenzhydryl)piperazin umgesetzt wird und anschließend die erhaltene Verbindung durch Reduktionsmittel, wie Metallhydride, reduziert wird.

4. Pharmazeutische Zusammensetzungen mit peripherer Calciumantagonisten-, antikonvulsiver und cerebraler eumetabolischer Wirksamkeit, welche als aktiven Wirkstoff die Verbindungen nach den Ansprüchen 1 bis 2 enthalten.

5. Pharmazeutische Zusammensetzungen nach Anspruch 4 in Form von Kapseln, Tabletten, Dragees, Sirups, Lösungen, Granulaten, Pastillen, Phiolen.

**Patentansprüche für den Vertragsstaat: AT**

1. Verfahren zur Herstellung von 1-(4,4'-Difluorbenzhydryl)-4-(3,4-dimethoxycinnamyl)piperazin der allgemeinen Formel I

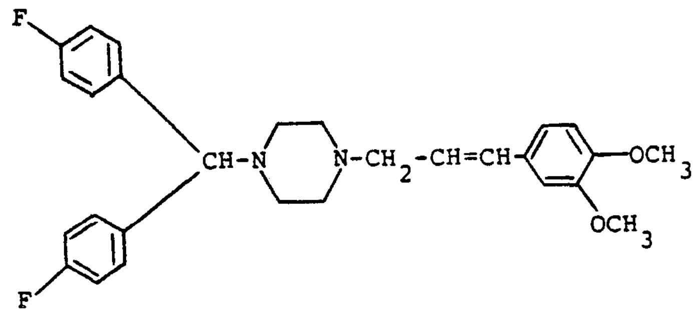

(I)

dadurch gekennzeichnet, daß ein reaktives Derivat von 3,4-Dimethoxyzimtsäure mit 1-(4,4'-Difluorbenzhydryl)piperazin umgesetzt wird und anschließend die erhaltene Verbindung durch Reduktionsmittel reduziert wird.

2. Verfahren nach Anspruch 1, dadurch gekennzeichnet, daß die Umsetzung in Gegenwart einer organischen tertiären Base durchgeführt wird.

3. Verfahren nach Anspruch 1, dadurch gekennzeichnet, daß die Reduktion durch Metallhydride wie Lithium-Aluminiumhydrid oder Natriumborohydrid durchgeführt wird.

4. Verfahren zur Herstellung von 1-(4,4'-Difluorbenzhydryl)-4-(3,4-dimethoxycinnamyl)-piperazin, dadurch gekennzeichnet, daß ein 3,4-Dimethoxycinnamylhalogenid mit 1-(4,4'-Difluorbenzhydryl)piperazin in Gegenwart einer organischen tertiären Base umgesetzt wird.

**Revendications pour les Etats contractants: BE CH DE FR GB IT LI LU NL SE**

1. 1-(4,4'-difluorbenzhydryl)-4-(3,4-diméthoxycinnamyl)pipérazine de formule générale:

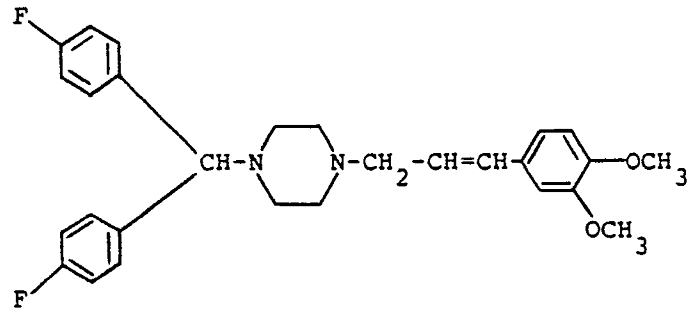

(I)

et ses sels acceptables pharmaceutiquement avec des acides organiques ou inorganiques.

2. En tant que composé selon la revendication 1, dihydrochlorure de 1-(4,4'-difluorobenzhydryl)-4-(3,4-diméthoxycinnamyl)pipérazine.

3. Un procédé pour la préparation des composés selon les revendications 1 ou 2, caractérisé en ce que l'on fait réagir un dérivé réactif de l'acide 3,4-diméthoxycinnamique avec la 1-(4,4'-difluorobenzhydryl)piperazine et en ce que l'on réduit ensuite le composé résultant au moyen d'agents réducteurs tels que des hydrures métalliques.

4. Compositions pharmaceutiques ayant une activité eumétabolique anticonvulsive et cérébrale, antagoniste de calcium périphérique contenant en tant que principle actif les composés selon les revendications 1 ou 2.

5. Compositions pharmaceutiques selon la revendication 4, en forme de capsules, tablettes, dragées, sirops, solutions, granulés, pastilles, ampoules.

**Revendication pour l'Etat contractant: AT**

1. Procédé pour la préparation de la 1-(4,4'-difluorbenzhydryl)-4-(3,4-diméthoxycinnamyl)pipérazine de formule générale:

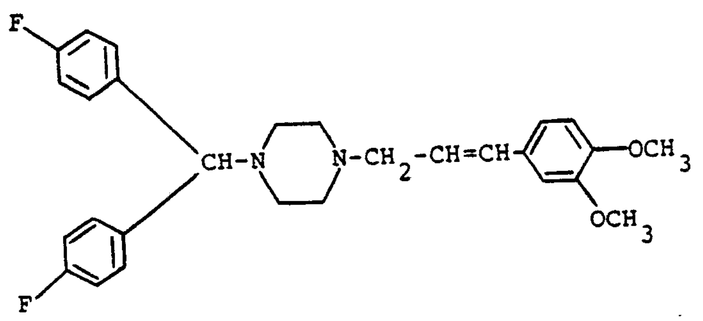

(I)

caractérisé en ce que l'on fait réagir un dérivé réactif de l'acide 3,4-diméthoxycinnamique avec la 1-(4,4'-difluorobenzhydryl)piperazine et en ce que l'on réduit ensuite le composé résultant au moyen d'agents réducteurs.

2. Procédé selon la revendication 1, caractérisé en ce que la réaction est effectuée en présence d'une base organique tertiaire.

3. Procédé selon la revendication 1, caractérisé en ce que la réduction est effectuée an moyen d'hydrures métalliques, tels que l'hydrure de lithium et aluminium ou le borohydrure de sodium.

4. Procédé pour la préparation de la 1-(4,4'-difluorobenzhydryl)-4-(3,4-diméthoxycinnamyl)pipérazine caractérisé en ce que l'on fait réagir un halogenure de 3,4-diméthoxycinnamyle avee 1-(4,4'-difluorobenzhydryl)-pipérazine en présence d'une base organique tertiaire.